Europäisches Patentamt

European Patent Office

Office européen des brevets

(19)

(11) Publication number: **0 320 095**
**A2**

## (12) EUROPEAN PATENT APPLICATION

(21) Application number: **88309621.6**

(22) Date of filing: **13.10.88**

(51) Int. Cl.4: **C12N 9/02 , G01N 33/72**

(30) Priority: **10.12.87 US 130921**
**01.07.88 US 214354**

(43) Date of publication of application:
**14.06.89 Bulletin 89/24**

(84) Designated Contracting States:
**CH DE FR GB IT LI**

(71) Applicant: **HEALTH & RESEARCH SERVICES INC.**
**100 International Boulevard**
**Etobicoke Ontario M9W 6J6(CA)**

(72) Inventor: **Wu, Tai-Wing**
**28 Admiral Road Unit No. 3**
**Toronto Ontario, M5R 2L5(CA)**

(74) Representative: **Paget, Hugh Charles Edward et al**
**MEWBURN ELLIS & CO. 2/3 Cursitor Street**
**London EC4A 1BQ(GB)**

(54) **Bilirubin degrading enzymes.**

(57) A bilirubin-degrading enzyme preparation obtained from higher plant sources of the genus Citrus is described. Exemplary sources include lemon, lime, orange, tangerine, tangelo, and grapefruit. Assay compositions, analytical elements and methods for using the enzyme are also described.

EP 0 320 095 A2

# BILIRUBIN DEGRADING ENZYMES

The invention relates to a bilirubin-degrading enzyme preparation obtained from plant sources.

The invention also relates to the use of such preparations and assay compositions, analytical elements and methods for using the enzymes.

Unconjugated bilirubin (Bu) is a catabolite of heme in mammals. It is well known that Bu is metabolized, principally in the liver (less so in the kidney, spleen and bone marrow), where two enzymes catalyze the addition of a sugar (chiefly glucuronic acid) onto either one or both of the propionic acid side chains of the Bu tetrapyrrole (R. Schmid and A. F. McDonagh in The Porphyrins, ed. D. Dolphin, Academic Press, New York, p258, 1979 and B. H. Billing Gut 19, p481, 1978). The resulting sugar conjugates, collectively termed conjugated bilirubin or Bc are then excreted into bile and from there, to the intestines, where further bacterial degradations of Bc occur and the products are excreted in the feces and via the urine. In newborns, in patients deficient in the liver enzymes that make Bc, and in hemolytic diseases, Bu is elevated in the blood. Bu is extremely apolar and neurotoxic and poses a special threat to neonates because elevated Bu can cross the blood-brain barrier to inflict irreversible brain damage or death (G. B. Odell in Neonatal Hyperbilirubinemia, Grune and Stratton, New York, 1980)

The presence of bilirubin degrading enzymes has been reported previously. In 1969, Brodersen and Bartels suggested the presence of a Bu-oxidizing enzyme of low specific activity from guinea pig brain that produces among others, biliverdin and yellow diazo-negative polar pigments (Europ J. Biochem 10, p.468). Yokosuka and Billing (Clin. Sci. 58, p.13 1980) later found a similar activity in the liver of Sprague-Dawley rats. More recently the same group reported that this enzyme activity in liver was suppressed by a cytosolic inhibitor that can be removed by subcellular fractionation (R. Cardenas-Vazquez et al, Biochem. J. 236, p.625, 1986). In 1980, the present inventor described a bilirubin-degrading enzyme from various fungi, and illustrated the activity in edible mushrooms (Agaricus bisporus) (U.S. Patent No. 4,211,844, issued July 8th, 1980). Subsequently, Murao and Tanaka, Agric. Biol. Chem. 45, p.2383, 1980 and Agric. Biol. Chem. 46, p2499, 1982 purified a similar enzyme which they also called bilirubin oxidase or BOD from the fungus Myrothecium verucarria MT-1.

The present inventor has also described an enzyme with bilirubin-degrading activity from various plant sources of the families Solanaceae, Musaceae and Ciliaceae. (U.S. Application No. 528,486). The enzyme was found to have a specific activity of up to several times higher than the specificity of the fungal enzyme described in U.S. Patent, 4,211,844. The bilirubin-specific enzyme derived from these plant families was found to degrade bilirubin at a pH in the range of from about 6 to about 10 and a temperature in the range from about 20° to 50° C.

There are a number of practical considerations of a bilirubin-degrading enzyme: (a) The enzyme can be used for the quantitative analysis of bilirubins from body fluids (eg. blood, urine, CSF), which is important in the differential diagnosis of jaundice and in monitoring liver transplantation patients (T.W.WU, Clin. Biochem 17, p.221, 1984 and T. W. Wu, Labmedica, 2; p.15, 1987); (b) It can also be used in removing bilirubin induced (spectral and/or chemical) interferences from other important clinical biochemical tests such as blood glucose, creatinine, cholesterol, triglycerides, uric acid, oxalate, lactate, and enzymes, for example, CK, ALT, AST, and GGT; (c) It can be used to remove neurotoxic bilirubin from the blood of jaundiced newborns to ward off possible brain damage or death by means of for example, an extracorporeal device/circuit containing immobilized bilirubin degrading enzyme. The current commonly used treatments of phototherapy and exchange transfusion have their own recognized risks. (Odell, supra). In preliminary studies, Lavin et al (Science, 230, p.543, 1985) reported promising results with a fungal enzyme-linked bioreactor connected extracorporeally to the chronically jaundiced Gunn rats. They found that over 90% of Bu was degraded in one pass. However, the fungal enzyme available for such manipulations is relatively expensive, varies in purity from batch to batch, and has less than ideal storage or in-bioreactor stability.

The present invention provides a novel bilirubinspecific, enzyme preparation comprising an enzyme obtained from certain higher plants, namely plants of the genus Citrus. It is the only bilirubin-degrading enzyme hitherto reported from this genus of higher plants in Kingdom Thallophyta. The plants of the families Solanaceae, Musaceae and Liliaceae from which the present inventor obtained a bilirubin-specific enzyme which is described in U.S. Patent Application No. 528,486, differ both biochemically and taxonomically from the higher plants in Kingdon Thallophyta. The enzyme preparation of the present invention has been found to be substantially more active than most other known bilirubin-degrading enzymes and it exhibits very high specificity for bilirubin. Most unexpectedly the enzyme preparation of this invention gives a product absorption spectra in solution that has hitherto never been reported and the enzyme preparation has been found to be extremely thermostable. Furthermore, the plants of the genus Citrus, particularly the peel of these plants which is generally discarded, are readily available inexpensive enzyme sources compared to the fungal and rat liver enzymes. In view of the above, the enzyme preparation of this invention obtained from the plants of the citrus family is highly suited for the commercial and medical utilities discussed above.

In accordance with one specific embodiment of the present invention, an enzyme preparation derived from lime (Citrus aurantifolia) was found to have a specific activity higher than that of any known bilirubin degrading enzyme in the crude state. The lime enzyme preparation at pH 5.0 also degraded a synthetic tauro-conjugate of bilirubin Bc isolated from humans and delta bilirubin. There is no single enzyme hitherto known in the art which is specific for delta bilirubin, which is measured by subtraction or by HPLC methods. The lime enzyme preparation of the present invention can accordingly be used in the separation of bilirubin at acidic pH. The lime enzyme preparation has many practical advantages for use in bilirubin assays since it is highly active, specific, stable and inexpensive.

Broadly stated, the present invention provides, a bilirubin-specific enzyme preparation comprising an enzyme derived from a plant of the genus Citrus which in the presence of a bilirubin containing aqueous liquid degrades bilirubin. An especially preferred embodiment of this invention provides a bilirubin-specific enzyme preparation, which does not generate hydrogen peroxide or biliverdin as a stoichiometric end product. A further preferred bilirubin-enzyme preparation according to the invention degrades about 0.022 - 0.069 moles of unconjugated bilirubin per minute per milligram of protein in the presence of a bilirubin-containing aqueous liquid having a pH of about 7.5 and a temperature of about 25°C.

The novel enzyme preparation of this invention can be used to remove bilirubin from a biological fluid such as whole blood or serum, when bilirubin is present in a detrimental amount. The enzyme preparation can also be used in an assay composition for the determination of an analyte other than bilirubin in an aqueous liquid sample containing bilirubin. Such an assay composition comprising an interactive composition and the enzyme preparation described hereinabove. The enzyme thereby degrades bilirubin and reduces its interference with the assay.

The invention also provides an analytical element for detecting an analyte (e.g. bilirubin) comprising the bilirubin-specific enzyme preparation described above. In a preferred embodiment, this element includes a support and a reagent zone containing the bilirubin-specific enzyme.

The invention further provides a method for the degradation of bilirubin in an aqueous liquid sample comprising contacting the liquid sample with a bilirubin-specific enzyme preparation to degrade bilirubin. Such enzyme preparation contains an enzyme derived from a plant of the genus Citrus which, in the presence of a bilirubin-containing aqueous liquid degrades bilirubin.

Preferred enzyme preparations are those which do not generate hydrogen peroxide or biliverdin as a stoichiometric end product. Particularly preferred preparations are those that degrade about 0.022-0.069 micromoles unconjugated bilirubin per minute per milligram of protein in the presence of a bilirubin-containing aqueous liquid having a pH of about 7.5 and a temperature of about 25°C.

One embodiment of this method for the degradation of bilirubin in an aqueous liquid sample is a method for the determination of bilirubin in an aqueous liquid sample comprising contacting the liquid sample with bilirubin-specific enzyme preparation to interact bilirubin with the enzyme preparation to produce a detectable change.

A second embodiment of the bilirubin-degrading method described above, is a method for the determination of an analyte other than bilirubin in an aqueous liquid sample comprising treating the liquid sample with bilirubin-specific preparation before or while the sample containing the analyte is treated with an interactive composition which produces a detectable change corresponding to the presence and/or amount of the analyte.

FIG. 1 is a graphical plot illustrating the absorption spectra of reaction product of the bilirubin-specific enzyme preparation of this invention (1B) and a graphical plot illustrating the absortion spectra of bilirubin alone (1A):

FIG. 2 is a graphical plot illustrating the change in bilirubin degradation velocity in response to the change in pH of the medium for incubating the bilirubin-specific enzyme preparation of this invention with bilirubin;

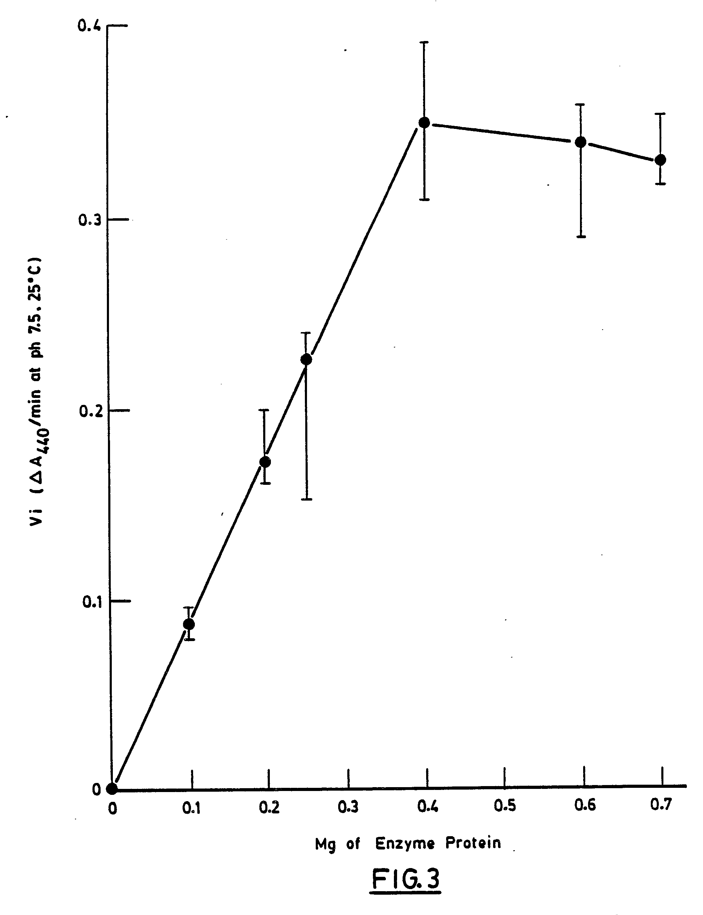

FIG. 3 is a graphical plot illustrating the change in bilirubin degradation velocity in response to the change in the amount of the bilirubin-specific enzyme preparation of this invention;

FIG. 4 is a graphical plot illustrating the kinetics of bilirubin degradation by the bilirubin-specific enzyme preparation of this invention in response to the change in the amount of bilirubin in the sample being assayed;

FIG. 5 is a graphical plot illustrating the thermostability of the bilirubin-specific enzyme preparation of this invention( ● ); and bilirubin oxidase from Myrothecium verucarria MT-1(o).

FIG. 6 is a graphical plot illustrating the change in bilirubin degradation velocity of the bilirubin-specific enzyme of this invention in response to changes in cholic acid concentration.

In accordance with the embodiments shown herein, a highly active, specific and stable bilirubin degrading enzyme from the genus Citrus has been demonstrated. It has been unexpectedly found that the reaction product of the enzyme preparation has an absorption maximum over 500 to 507 nanograms with no evidence of biliverdin either as an intermediate or a stoichiometric end product. The enzyme preparation degrades 0.022 to 0.069 micromoles of unconjugated bilirubin per minute per milligram of protein at pH 7.5 and 25° C. This level of specific activity is at least 10 and 30 folds higher respectively than those reported for other bilirubin-degrading enzymes from the edible mushroom Agaricus bisporus and from rat liver (Table 1). The enzyme has a broad pH optimum between pH 7.4 and 7.8 which is close to the human physiological system making the enzyme preparation particularly advantageous as a therapeutic and diagnostic agent. The enzyme preparation of the invention has a Km of about 50 to 270 micromoles Bu indicating that it exhibits very high specificity for bilirubin. The enzyme is more specific for bilirubin than the other bilirubin-degrading enzymes from the edible mushroom and from rat liver (Table 1). The enzyme preparation also has remarkable stability retaining 90 to 100% activity after heating at about 100° C and at a pH of 7.5 for about 30 minutes. Under similar conditions, the bilirubin oxidases from fungus and rat liver lost over 50 to 80%

Table I

| | Specific Activity ($\mu$ Moles/min/mg) | Km |
|---|---|---|
| Citrus Enzyme | 0.022-0.069 | $5\text{-}27 \times 10^{-5}$ M |
| * Fungal Enzyme | 0.0015-0.005 | $15.4 \times 10^{-4}$ M |
| ** Rat Liver | 0.00157 | $13.6 \times 10^{-5}$ M |

* Data from U.S. Patent No. 4,211,844. One unit of enzyme activity was defined as that amount of enzyme that catalyzes the conversion of one $\mu$mole of Bu per minute at pH 7.45±0.05 and 22° -25° C. Specific activity (S.A.) is the acti ity unit divided by mg of protein used per assay. S.A. reported is for the crude fungal enzyme preparation. The Km is disclosed in column 13, line 60.
** Data from Cardenas-Vazquez et al. Biochem. J. 236, p. 625, 1986. The specific activity was determined at a temperature of 37° C. It would be expected to those skilled in the art that the specific activity would have been lower at 5° C.

of their starting activities.

The bilirubin-specific enzymes useful in these preparations can be extracted from plants of the genus Citrus. Exemplary species of such plants include oranges (Citrus sinensis), lemons (Citrus limon), limes (Citrus aurantifolia), tangelos (a hybrid of the Dancy tangerine and grapefruit), tangerines (Citrus reticulata) and grapefruits (Citrus paradisi). The enzymes which are preferred are those derived from limes, tangerines, tangelos and oranges, particularly preferred are limes and oranges.

The enzymes can be extracted by blending the pulp or peel of the desired plant with an appropriate liquid to form a homogenous aqueous mixture. Such blending is conveniently carried out by homogenizing the pulp or peel in 5 volumes of distilled water using an Osterizer (Sunbeam, Toronto, Ontario) as a homogenization device; other equivalent blending techniques can also be employed. The aqueous mixtures may be filtered for example, through cheesecloth to ensure homogeneity. Insoluble solids can be separated from the homogenous mixture, for example, by centrifuging. Especially high bilirubin degrading activity was observed when the enzyme was extracted from the peel.

Bilirubin exhibits a characteristic absorption peak ($\lambda$ max) at about 440 nm of the electromagnetic spectrum. When the enzyme preparation is incubated with a bilirubin containing aqueous liquid under suitable pH and temperature conditions the absorbance or absorption density at $\lambda$ max decreases rapidly. This density decrease is a detectable change and can be monitored against a reference sample which lacks bilirubin. In this manner an aqueous liquid sample can be assayed for bilirubin.

In accordance with one embodiment of the invention the enzyme preparation is used to degrade bilirubin in a biological fluid, e.g. whole blood, when bilirubin is present in that fluid in an excessive amount. The fluid can be passed through a filter device (either implanted in a patient or ex vivo) containing the enzyme preparation. A bilirubin binding material can be used in combination with the enzyme preparation to remove undegraded bilirubin or the products of bilirubin degradation. Examples of such material are albumin or a mordant such as that described in U.S. Patent No. 4,338,095 (issued July 6, 1982 to T.W. Wu).

In another embodiment of the invention a method for the determination of bilirubin in an aqueous liquid sample, such as a biological fluid (e.g. whole blood, plasma, sera, lymph, bile, urine, spinal fluid, sputum, sweat as well as stool and secretions of humans or animals)is disclosed.

The method for the determination of bilirubin in aqueous liquid comprises:

(a) contacting an aqueous liquid sample with the bilirubin-specific enzyme preparation of this invention at a pH and temperature effective for the enzyme preparation to interact with bilirubin and produce a detectable change corresponding to the presence and/or concentration of bilirubin; and

(b) detecting such a change.

A further embodiment of the invention provides a method to reduce bilirubin as an interferent in assays for analytes other than bilirubin, for example, biochemical assays of uric acid, glucose, cholesterol, and creatinine. Such assays may be carried out with assay compositions comprising a bilirubin-specific enzyme preparation of this invention and interactive compositions which, when they interact with the analyte, produce a detectable change of some type (e.g. colorimetric, fluorometric, potentiometric etc.). Any suitable interactive composition can be used with an enzyme preparation of this invention. Examples of interactive compositions include enzyme linked hydrogen peroxide detection systems, enzyme linked NAD-NADH detection systems, redox reactions, hydrolase reactions and others known in the clinical chemistry art.

Useful interactive compositions in assays of an analyte other than bilirubin can be any compositions capable of physical, electrical, chemical or other interaction with the analytes which lead to a detectable change, for example, an absorbance shift or a change in absorption density, which can be related to the presence and/or amount of the analyte.

The method of reducing bilirubin as an interferent in aqueous samples to be assayed for analytes other than bilirubin comprises:

(a) treating the liquid sample with an interactive composition for the analyte of interest to produce a detectable change e.g. a color change, corresponding to the presence and/or amount of the analyte;

(b) prior to or during treatment (a), contacting the liquid sample with an enzyme preparation of this invention thereby degrading bilirubin and reducing its potential for interference with a detectable change produced in (a); and

(c) detecting the change produced in (a).

The enzyme preparations of the invention are particularly effective at a pH of about 7.5 and a temperature of about 25°C but the enzyme preparations can be used over wider pH and temperature ranges. The enzyme preparations provide useful bilirubin degrading activity over a pH range from about 7.4 to 7.8 and a temperature range from about 15 to about 100°C.

A buffer may be used with the enzyme preparation of this invention to maintain the pH within the pH range effective for the interaction of the enzyme preparation with bilirubin. A variety of buffers can be used with the enzyme preparation and some appropriate buffers are described, for example, by Good in Biochemistry, 5, p.467, 1966.

The amount of the enzyme preparation to be used in the methods of the invention varies with the bilirubin concentration of the liquid sample to be assayed and the activity of the particular enzyme preparation. Generally, the enzyme preparation is used in an amount in the range of from about 0.02 to about 0.1mg, and preferably in the range from about 0.05 to about 0.1mg. This assumes that each miligram of enzyme preparation has a minimum activity level for bilirubin of at least 0.022 micromoles of bilirubin per minute of protein as determined in aqueous liquid at a pH of about 7.5 and a temperature of about 25°C. When using an enzyme preparation having higher activity a smaller amount of enzyme preparation can be used.

When an enzyme preparation of this invention is used to reduce bilirubin as an interferent in assays for analytes other than bilirubin the interactive composition which is employed must itself be non-interfering with respect to the enzyme preparation. For example, if the interactive composition contains a hydrogen peroxide detection composition, it would clearly be inappropriate to use an enzyme preparation which itself generates hydrogen peroxide. However, this particular problem is avoided because the enzyme preparation of this invention can be used to degrade bilirubin without generation of hydrogen peroxide.

The detectable change produced in the methods of the invention can be detected by a number of means. For example, a suitable detection device capable of detecting a change in absorption density, a change of fluorescent or radioactive emission or a shift in the absorbance of a characteristic λ max can be employed.

The enzyme preparation, assay composition, and methods of the invention can be employed in both solution ("wet chemistry') or dry element ("dry chemistry') assays. These assays and the elements used in the assays are described in U.S. Patent No.4,701,411 which is incorporated herein by reference. The description is essentially set out below for completeness.

In solution assays, the assay is carried out entirely in a liquid medium and the enzyme preparation or assay composition containing it is employed as a liquid reagent. In such a case, the enzyme preparation or assay composition is mixed with the aqueous liquid sample to be assayed at a suitable pH and temperature. When used in such assays the bilirubin-specific enzyme preparation is preferably present in an amount in the range of from about 0.1 to 0.2 mg enzyme protein. The solution assay is preferably carried out at a pH in the range from about 7.4 to 7.8 and at a temperature in the range of from about 25°C to 37°C.

In dry element assays, the enzyme preparation or assay composition can be incorporated, by imbibition, impregnation, coating or other suitable techniques into a suitable location of a suitable analytical element. For example, the enzyme preparation or assay composition can be incorporated into a reagent layer of a dip-and-read fibrous test strip or a reagent layer of a non-fibrous multilayer element, such as that described in U.S. Patent 3,992,158 (issued November 16, 1976 to Przybylowicz et al), which is incorporated herein by reference. The methods of this invention are practiced by contacting (e.g. spotting) the dry element with the aqueous liquid sample to be assayed. Appropriate apparatus is used to measure any detectable change which occurs within the element. The enzyme preparation or assay composition is present in the dry element as a dried residue, for example, as a freeze-dried powder or dried residue of a coating composition.

The bilirubin-specific enzyme should be coated in a layer physically distinct from other layers of the dry-element so that mixing with the incoming fluid is obtained only upon wetting the element with serum or biological fluid. Also, the bilirubin-specific enzyme should be placed close to the zone of the dry element in contact with incoming fluid so it can act on bilirubin first. Any serum proteins that might influence its activity should be retained in another layer or prevented from contacting the enzyme layer, for example, by a filtration mechanism. A sample coverage of the bilirubin-specific enzyme is between about 0.5 to 2.0 mg per ft$^2$.

Generally, the analytical elements of this invention have at least one reagent zone containing the enzyme preparation or assay composition of this invention. Self-supporting zones, that is, zones composed of materials rigid enough to maintain their integrity can be used, but preferably the zones are carried on a support. The support can be of any suitable dimensionally stable, and preferably, transparent (i.e. radiation transmissive) material which transmits electromagnetic radiation of a wavelength between about 200 and 900 nm. Examples of support materials are polystyrenes, polyesters (e.g. Poly(ethyleneterephthalate)), polycarbonates, cellulose esters, etc. The element can have more than one zone, some or all of the zones, containing reagents. The zones are in fluid contact with each other so that fluids can be transported between adjacent zones. The zones are preferably separate coated layers, although one or more zones can be in a single layer of an element. Dry element formats and materials to make the formats are known and are described, for example, in U.S. Patent 3,992,158 noted hereinabove; U.S. Patents 4,042,335 (issued August 16, 1977 to Clement); 4,144,306 (issued March 13, 1979 to Figueras); 4,132,528 (issued January 2, 1979 to Eikenberry et al); 4,050,898 (issued September 27, 1977 to Goffe et al); 4,258 001 (issued March 24, 1981 to Pierce et al); and Reissue 30,267 (reissued May 6, 1980 to Bruschi) the disclosures of which are incorporated herein by reference.

The reagent zones of these elements can also act as spreading zones. The element can additionally have or alternatively have one or more separate spreading zones. A spreading zone is generally a layer which can accept a liquid sample. When the liquid sample is applied directly to the layer or provided to it from a layer or layers in fluid contact with it, the solvent or dispersion medium of the sample is distributed such that a uniform apparent concentration of the sample is provided at the surface of the spreading layer facing the adjacent reagent layer. Examples of materials that can be used for preparing spreading zones are described, for example, in U. S. Patents 3,992,158 and 4,258,001, noted hereinabove; and 4,292,272 (issued September 29, 1981 to Kitajima et al); and U.K. Patent Application 2,052,057 (published January 21, 1981). The spreading zone can be made of either fibrous or non-fibrous materials, or both. The spreading zone is preferably an isotropically porous spreading layer as described in U. S. Patent 3,992,158, noted hereinabove.

Other components may be present in one or more zones (eg. reagent, spreading, subbing barrier or registration) of the elements of this invention. Examples of such other components are surfactants, enzyme activators, binders (generally hydrophilic natural or synthetic colloids or polymers), hardeners, dyes, solvents, etc. These components are present in amounts known in the clinical chemistry art.

In accordance with the present invention, a variety of different elements, depending on the method of assay, can be prepared. Elements can be configured in a variety of forms, for example, elongated tapes of any desired width, sheets or smaller chips.

The analytical methods of this invention can be manual or automated. In general, to determine the amount of bilirubin or other analyte in a liquid the element is taken from a supply roll, chip packet or other source and physically contacted with a sample of the liquid. The element and liquid can be contacted in any suitable manner, for example, dipping or immersing the element into the sample, or, preferably, by spotting the element by hand or machine with a drop of the sample by pipette or another suitable dispensing means. The element is then exposed to any conditioning, such as incubation, heating or the like, that may be desirable to quicken or otherwise facilitate obtaining any test result. Any analyte or bilirubin present will react and produce a detectable change. The detectable change is quantifiable by passing the element through a zone in which suitable apparatus for detection is provided.

Examples of detection means which can be used are reflection or transmissive colorimetric spectrophotometry, fluorescence spectrophotometry, radiometry, chemiluminescence, enzyme labeling, measurement of enthalpy changes and the like.

The examples which follow illustrate the invention. The following information is common to each example:

Protein concentration was determined for quick reference by assuming that a 1 mg/ml of protein solution had an absorbance of 1 at 280nm. For more accurate determination of protein concentration, as used in specific activity calculations, the method of Lowry et al (J. Biol. Chem. 193, p. 265, 1951) was used.

The bilirubin used is a product of Sigma Chemical Co., St. Louis, Missouri. The bilirubin (BOD) from Myrothecium verucarria MT-1 was a product of Amano Co., Osaka, while the rat liver enzyme was prepared as described by Cardenas-Vazquez et al (Biochem. J. 236, p. 625, 1986).

Example 1

Extraction of the bilirubin degrading enzyme from oranges

Sunkist oranges from California were purchased from local groceries at various times of the year. The whole fruits were washed briefly under tap water, blotted on clean paper towels, then peeled with a sharp knife. The peels were minced into smaller pieces of, for example, 1 cm long X 1 cm wide, then homogenized mechanically in 5 volumes of ice chilled distilled water. The homogenization was done with four to five 30-second blasts in an osterizer (Sunbeam, Toronto, Ontario) at top speed setting interspersed with 20-second immersions of the homogenization vessel in an ice bath. The homogenate was filtered through two layers of cheesecloth and centrifuged at 9,000 X g for 20 minutes at 0 to 4 °C whereupon three distinct phases became apparent. There was a bottom pellet layer, an intermediate clear supernatant, and a top layer of "floating" material. As shown in example 2 the top layer appeared to contain most of the crude enzyme activity. The crude enzyme preparation was suspended in an equal volume of 0.05 M Tris-HCl buffer, pH 7.6 and frozen at -70°C in 1-ml aliquots. When thawed, the aliquots were used in the examples below. Despite the poor solubility of the crude enzyme, its aliquots within each batch contained closely similar activity units. The same overall protocol was essentially employed where the pulp was used separately from the peel except that the seeds were first removed, and the remaining pulp tissue was homogenized directly with three blasts in the mechanical homogenizer. Typically, the pulp homogenate separated, after centrifugation, into a yellowish supernatant (with variable degree of clarity) and a pellet fraction.

The methodology as described above was essentially used for extracting the enzyme from the limes, tangerines, tangelos, grapefruits and lemons.

Example 2

## Assay of bilirubin degrading activity

The unconjugated bilirubin (Bu) solution used in the assay of bilirubin degrading activity was prepared as follows: Stock solutions containing 340 M of Bu were prepared daily by dissolving 20 mg. of the solid (Sigma) in 0.33 ml of dimethylsulfoxide followed by addition of 0.67 ml of 0.1 M sodium carbonate. The solution was neutralized with 0.1 M HCl, and diluted to 100 ml with either sodium phosphate or Tris-HCl buffer (where indicated) at pH 7.5 -7.6 to give a bright red and clear solution. All dilutions were made in the same buffer as used to prepare the stock solution. The solutions were kept in dim light and preferably under a steady stream of nitrogen until just before use. Whenever there was the least sign of particulate matter in any Bu solution, the latter was discarded and a new solution made.

For routine assays, 1 ml of Bu stock solution prepared as described above was incubated at 25°C with 5 -10 microlitres of the crude enzyme obtained by the procedure described in Example 1. The change in absorbance at 440 nm was followed over three minutes in a Beckman Du-40 Spectrophotometer. Under otherwise identical conditions, the corresponding change in and absorbance for a Bu-only (ie minue enzyme) control solution was also monitored and subtracted from that in the enzyme reaction. The mean of two to four replicate determinations at each condition was generally reported. A unit of specific activity was defined as that amount of enzyme protein that degrades one μmole of Bu per minute at 25°C and 7.5.

Table II shows a summary of the specific activities obtained for more than 100 oranges obtained over a period of a year. Despite considerable variability in size, weight, fluid content and thickness of the crusts among oranges, the total Bu-degrading activity units were found with few exceptions to be roughly evenly distributed between the crude homogenates of the pulp and peel. Generally 80-90% of the total activity in the peel homogenate was recovered as the sum of activities in the three fractions obtained after centrifugation, with the top layer accounting for 50-60% and the other two fractions about 20% each. With the pulp homogenate, however, centrifugation often resulted in a loss of total activity (of the order of 50-70%), and the resultant supernatant layer harbored 10-20 times the total activity found in the pellet layer. Although the bottom pelleted layers from both the peel and pulp appeared to have impressive specific activities of the enzyme (i.e. 0.020-0.035 μmoles/min/mg), these activities varied considerably from preparation to preparation and their estimates were subject to possible error since the fractions were extremely insoluble in aqueous media. The supernatants from both the pulp and peel had lower specific activities which also fluctuated from fruit to fruit (0.002-0.015 units). Accordingly, the top layer of the peel, (hereinafter referred to as the "orange peel enzyme preparation) with a more consistently high specific activity ranging between 0.022-0.058 units, was selected for further studies (see Examples 3-10).It is to be noted that the specific activity range of this top layer of the peel is at least 10 and 20 times higher respectively than the Bu-degrading enzymes reported from Agaricus bisporus (U.S. Patent No. 4,211,874 issued July 8, 1980) and from rat liver (R. Cardenas-Vazquez et al., Biochem. J. 236, p. 625, 1986).

TABLE II

| Fraction | Specific Activity ** |
|---|---|
| **Peel** | |
| Floating Material | 0.022 - 0.058 |
| Supernatant | 0.002 - 0.015 |
| Pellet | 0.020 - 0.035 |
| **Pulp** | |
| Supernatant | 0.002 - 0.015 |
| Pellet | 0.020 - 0.035 |

** Specific activity was determined by calculating the number of $\mu$moles of unconjugated bilirubin degraded per minute per mg at pH 7.5 and a temperature of 25°C.

Example 3

### Absorption Spectra of the Orange Peel Enzyme preparation

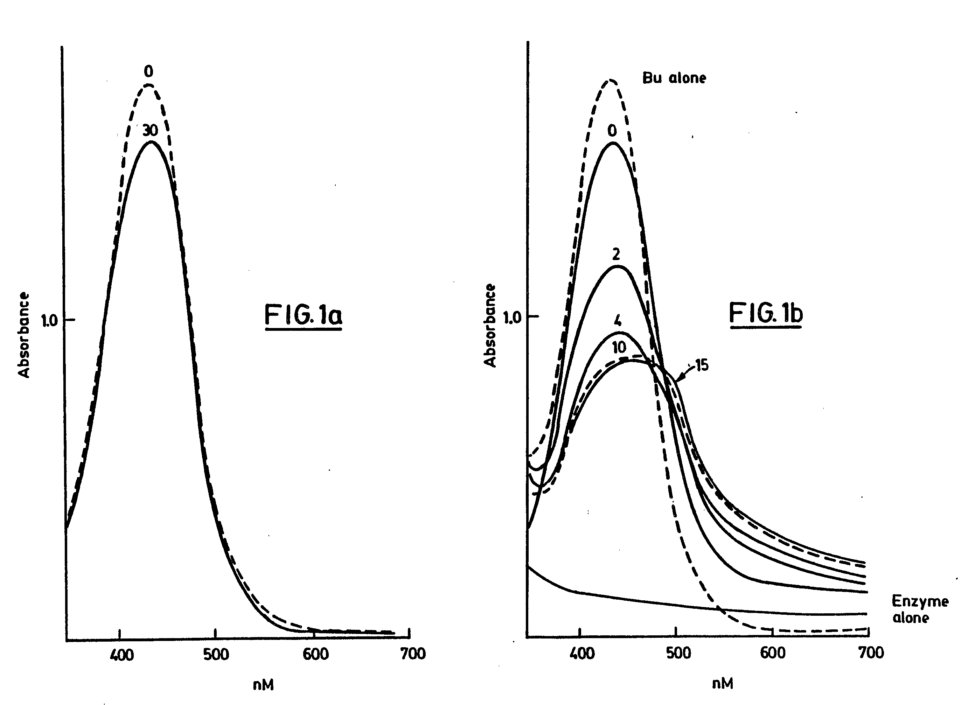

Approximately 0.1 mg of the orange peel enzyme preparation obtained by the procedure described in Example 1 was incubated with an aqueous solution containing approximately 2 mg/dL of bilirubin at pH 7.5 and 25°C. As illustrated in Figure 1, the absorbance of bilirubin at its 440 nm peak rapidly decreased with a concomitant rise in the 500-507 nm range. Although there was a general rise in the absorbance in the longer wavelengths (e.g. 520-700nm), there was no evidence of a distinct peak at 380 nm, which might have suggested biliverdin formation as an intermediate or an end product of the enzymic reaction. Under otherwise identical conditions, a solution of Bu alone moderately decreased in its peak absorbance at 440 nm with time (Figure 1A). There was no dramatic change in the overall spectrum of Bu alone.

Example 4

### Bilirubin-Degrading Activity as a Function of pH

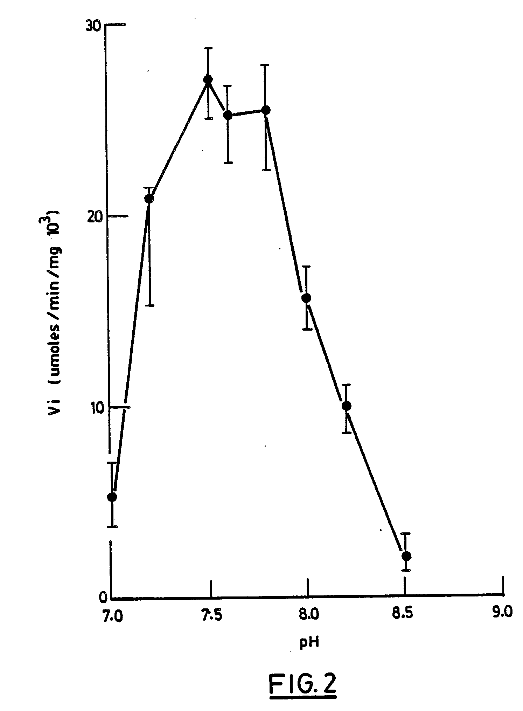

A series of enzyme mixtures were prepared, each containing orange peel enzyme preparations obtained by the procedure described in Example 1 and a different amount of buffer to provide a different pH level. All other assay conditions were as described in Example 2. Figure 2 shows that the enzyme exhibited a broad pH optimium between 7.4 to 7.8. The apparent pH maximum is at 7.8.

Example 5

### Bilirubin-Degrading Activity as a function of Orange Peel Enzyme Concentration

Different amounts of the orange peel enzyme preparation obtained by the procedure described in

9

example 1 were added to the Bu stock solution. Test conditions were the same as described in example 2. As illustrated in Figure 2, the initial velocity varied linearly with enzyme protein up to approximately 0.25 mg. Beyond this protein level, the velocity actually decreased with increasing amount of the crude enzyme. This suggested among others, that there might have been endogenous inhibitors or competing activities in the crude preparation.

Example 6

Bilirubin-Degrading Activity as a Function of Bilirubin Concentration

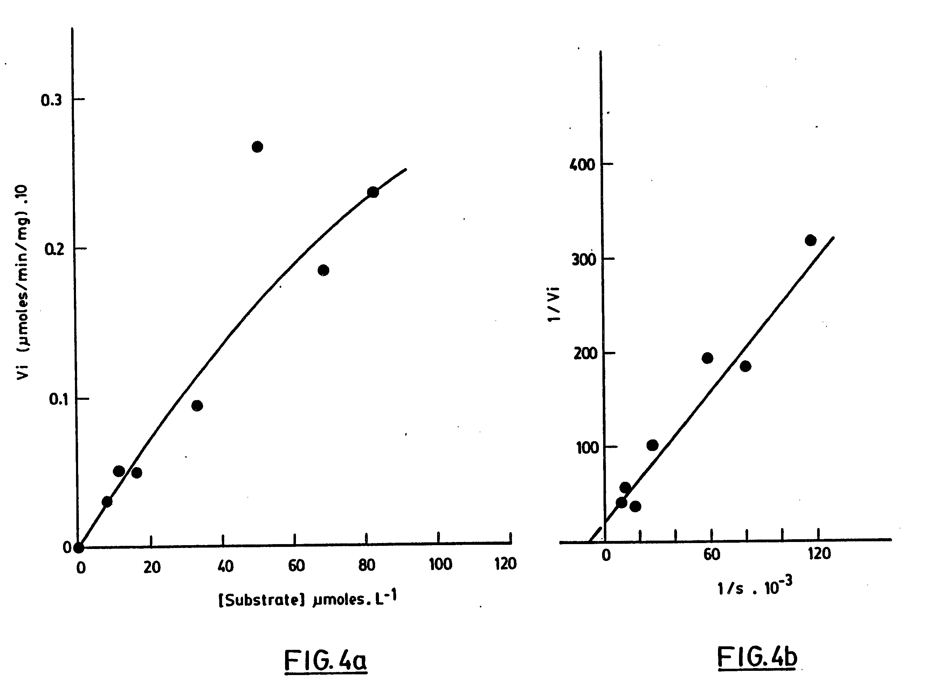

An aliquot of the orange peel enzyme preparation obtained by the procedure as described in example 1 was added to separate samples having concentrations of bilirubin ranging from about 0 up to 90 $\mu$M. Test conditions were the same as those described in Example 2. As illustrated in Figure 4, the enzyme followed Michaelis - Menten Kinetics, with an apparent Km of 50-100 $\mu$M Bu. (See Lineweaver & Burke, J.A.C.S., 56, p 658, 1934).

Example 7

Bilirubin-Degrading Activity as a Function of Temperature

The bilirubin-degrading assay as described in example 2 was carried out at pH 7.5 and at a temperature ranging from about 15 to 60° C. At pH 7.5 the enzyme had an apparent temperature optimum of 44° C.

Example 8

Stability of Orange Peel Enzyme

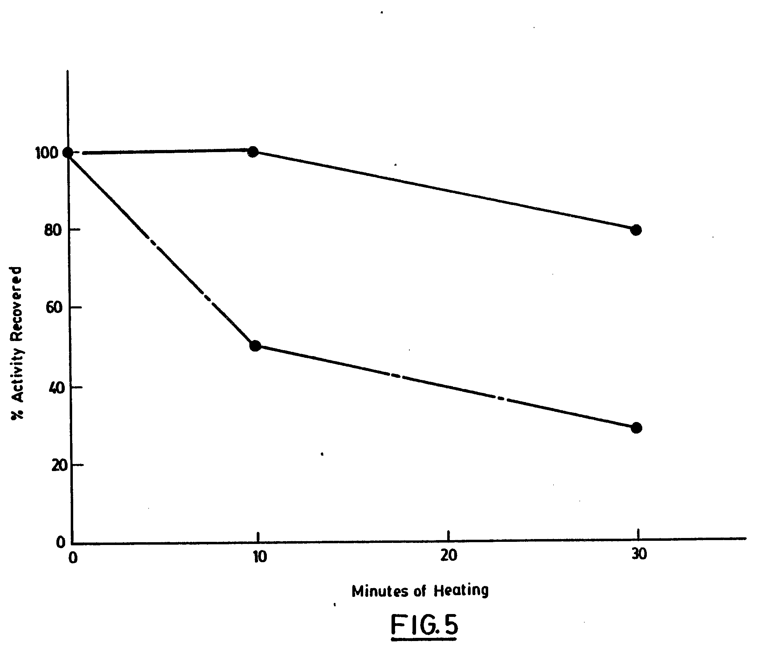

The orange peel enzyme preparation obtained by the procedure as described in example 1 was heated at 100° C and pH 7.5. As illustrated in Figure 5, the peel enzyme (●) retained 90-100% of its starting activity for one hour. Similar treatment of the bilirubin oxidase (BOD) from Myrothecium verucarria MT-1 (⊙) destroyed over 50-80% of the activities of the enzymes after 30 minutes. Thus, the peel enzyme was extremely thermostable when compared to the fungal and rat liver enzymes.

Example 9

Solubility of the Peel Enzyme

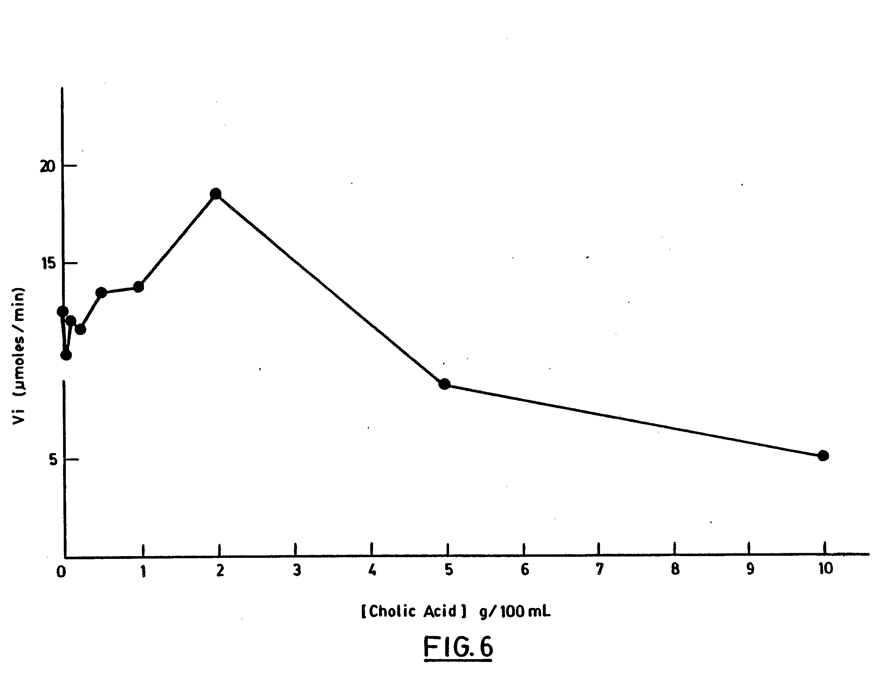

One of the unusual properties of the orange peel enzyme was its limited solubility in water. Numerous attempts had been made to solubilize it, including various organic solvents (e.g. propanol, acetone, ethanol), anionic, neutral, and cationic surfactants, as well as sonication and treatment with phospholipase. Of these, only cholic acid (at approximately 2% and above) was able to partially solubilize the activity. Fig. 6 shows the effect of varying levels of the surfactant on enzyme activity. Following treatment of the enzyme with cholic acid, between 20-30% of the resulting total activity remained in the supernatant after centrifugation at 100,000xg for 1-2 h at 4°C.

Example 10

Bilirubin-Degrading Activity of Peel Enzymes Obtained from other Citrus species

Bilirubin-specific peel enzyme preparations were obtained from tangerines, tangelos, grapefruits and lemons by the procedure as described in example 1. Bilirubin-degrading activity was determined in accordance with the assay procedure as described in example 2.

Results obtained from the peel enzymes extracted from the different Citrus species are shown in Table III. Based on the specific activity, the enzyme is located in descending order of specific activity in the peels of tangerine, tangelo, orange, grapefruit and lemon.

TABLE III

| Citrus Species | Specific Activity |
|---|---|
| tangerine | 0.05244 |
| tangelo | 0.0464 |
| orange | 0.0382 |
| grapefruit | 0.0292 |
| lemon | 0.02566 |

* (average of replicate determinations)

Example 11

Bilirubin-Degrading Activity of Peel Enzyme Obtained From Limes

Bilirubin-specific peel enzyme preparations were obtained from limes by the procedure as described in Example 1 and bilirubin degrading activity was determined in accordance with the assay procedure as described in Example 2.

The lime enzyme preparation degraded 0.035 to 0.069 $\mu$ moles unconjugated bilirubin per minute per mg of protein at pH 7.5 and 25°C and had a Km of 2.7 x $10^{-4}$M which is well within the range of Km's expected of a degradative enzyme. The pH optimum of the preparation was near 7.5 to 7.8 and the temperature optimum was 44°C.

Example 12

Effect of Citrus Enzyme on Human Hemoglobin, Biliverdin, Conjugated Bilirubin and Delta Bilirubin

Lime and orange enzyme preparations were obtained by the procedure as described in Example 1 and the bilirubin degrading assay as described in Example 2 was carried out in the presence of human hemoglobin (at 0.1, 1 and 2 g/dL respectively) biliverdin (2x crystallized at 1, 5 and 20 mg/dL respectively); conjugated bilirubin (mixtures of mono-and di-conjugates purified as per Wu et al., (Clin. Chem. 26:1241-1387, 1980 and Clin. Chem. 28:2398-2404, 1982) 1980) at 1, 5 and 10 mg/dL separately); or delta-bilirubin at 3 and 6 mg/dL) for up to 1 hour at pH 7.5 to 7.6. Human hemoglobin, biliverdin, conjugated bilirubin and delta-bilirubin, compounds that are the metabolic kith and kin to bilirubin were not affected noticeably by the enzyme preparation under normal assay conditions at pH 7.5 to 7.6 for up to 1 hour.

Significantly, at pH 5, the lime enzyme preparation, and less so the orange preparation, degraded a synthetic tauro-conjugate of bilirubin (molecular structure identified by Wu T.W. & Doumas, (Clin. Chem. 31: 1677, 1985) authentic bilirubin isolated from humans (Wu T.W. and Sullivan S.S. Clin. Chem. 28: 2398, 1982), and delta bilirubin (Vide, infra, 1982) indicating that the lime enzyme is useful for sub-fractionating bilirubins at acidic pH.

## Example 13

## Effects of the Citrus Enzyme on Routine Analysis of Serum Components

Biochemical assays of uric acid, glucose and cholesterol were carried out in a commercial calibrator matrix (Preciset) at two separate concentrations per analyte in the presence of a lime enzyme preparation obtained from limes by the procedure described in Example 1. Table IV shows that the presence of the lime enzyme at normal assay concentrations did not affect appreciably (i.e. 10-12% of the gravimetric value) the biochemical assays.

Table IV

| Hitachi Assays of Common Analytes in Artificial Matrix | | | |
|---|---|---|---|
| Analyte | No Enzyme | + Enzyme | + Enz./-Enz. x 100% |
| Uric Acid ($\mu$M) | 237 | 233 | 98.3 |
| | 713 | 636 | 89.2 |
| Glucose (mM) | 5.5 | 5.6 | 101.8 |
| | 22.2 | 20.9 | 94.1 |
| Cholesterol (mM) | 2.59 | 2.86 | 110.4 |
| | 10.34 | 10.91 | 105.5 |

To more closely reflect real serum situations, the biochemical assays described above were repeated using a pooled serum matrix, aliquots of which were supplemented with known amounts of urate and creatinine with/without the lime enzyme preparation. The cholesterol and glucose samples were real patient sera. Table V shows that the recovery for each analyte was over 97% of the assigned or known values even after the enzyme had been added (for at least 0.5 hour before analyses).

Table V

| Effect of Lime Enzyme on Blood Analyses in True Serum Matrix | | | |
|---|---|---|---|
| Analyte | Assayed (-Enzyme) | Assayed (+Enzyme) | % Recovery |
| cholesterol | 8.55 mM | 8.55 mM | 99.4 |
| | 2.88 mM | 2.83 mM | 99.5 |
| Urea | 18.5 mM | 19.6 mM | 105.9 |
| | 3.1 mM | 3.5 mM | 112.9 |
| Urate | 557 $\mu$M | 597 $\mu$M | 107.2 |
| | 259 $\mu$M | 279 $\mu$M | 107.7 |
| creatinine | 201 uM | 209 uM | 103.9 |
| | 94 uM | 91 uM | 97 |
| Triglyceride | 9.34 mM | 9.21 mM | 99 |
| | 0.76 mM | 0.81 mM | 106.6 |
| Glucose | 18 mM | 17.4 mM | 97 |
| | 4.5 mM | 4.4 mM | 98 |

The biochemical assays described above were also carried out using aliquots of sera pools, as obtained or supplemented with exogenous compounds so as to represent at least two distinct levels per analyte as shown in Table VI. The assays were carried out with or without an orange enzyme preparation obtained by the procedure described in Example 1. In particular, a normal assay level of orange enzyme preparation (10 microliters, accounting for ~100 $\mu$g protein) was added to each aliquot and incubated for 20 minutes at 37° C and the incubates were submitted for analyses on a Hitachi autoanalyzer. As controls, the aliquots not incubated with enzyme were supplemented with 10 $\mu$L of saline or a normal serum pool. The average of 2-3 replicate determinations are shown in Table VI. The results in Table VI show that the orange enzyme, like the lime enzyme, did not affect the normal biochemical tests of common serum analytes in a serum matrix.

Table VI

| Effect of Orange Enzyme on Hitachi Assays of Common Analytes in a Serum Matrix | | | |
|---|---|---|---|
| Analyte | -Enzyme | +Enzyme | % Recovery |
| Glucose | 18 mM | 18 mM | 100 |
| | 4.5 mM | 4.4 mM | 98 |
| Urate | 557 $\mu$M | 559 $\mu$M | 100 |
| | 259 $\mu$M | 269 $\mu$M | 104 |
| creatinin- e | 200 $\mu$M | 210 $\mu$M | 105 |
| | 100 $\mu$M | 95 $\mu$M | 95 |
| choleste- rol | 9.5 mM | 9.7 mM | 102 |
| | 2.2 mM | 2.05 mM | 93 |

13

Example 14

## Effect of Common Blood Components on the Citrus Enzyme Reaction

A. Lime Enzyme Reaction

The bilirubin-degrading assay as described in Example 2 was carried out using a lime enzyme preparation obtained as described in Example 1. Urate (at either 1.2 mM or 297 $\mu$M) and creatinine (88.4 and 176.8 $\mu$M) in a serum-based matrix had no effect on either the rate or extent of bilirubin degradation by the Citrus enzyme.

Table VII shows the effect of 3 levels of glucose in a serum matrix on the extent of reaction (after 30 min.) by the enzyme. It is noted that the bilirubin was extensively degraded whenever enzyme was added, regardless of how much or whether glucose was there or not.

Table VII

| Effect of Serum Glucose on Extent of Lime Enzyme Reaction | | | | | | |
|---|---|---|---|---|---|---|
| Permutation | | | Ektachem | | Hitachi | |
| [Glucose] mM | [Bu] $\mu$M | Enzyme | [Glu] mM | [Bu] $\mu$M | [Glu] mM | [Bu] $\mu$M |
| 33.3 | 0 | | 34.5 / 35.7 | <2 / <2 | 35.1 | -1 |
| 11.1 | 0 | | 10.8 / 11.3 | <2 / <2 | 12.2 | 1 |
| 5.5 | 0 | | 5.5 / 5.7 | <2 / <2 | 6.2 | 0 |
| 33.3 | 340 | | 36.2 / 37.0 | 320 / 326 | 30.1 / 30.1 | 87 / 108 |
| 11.1 | 340 | | 11.1 / 11.4 | 322 / 303 | 11.5 | 108 |
| 5.5 | 340 | | 5.8 / 5.9 | 325 / 310 | 5.6 | 110 |
| 33.3 | 15 | | 35.4 / 36.7 | 15 / 9 | 33.5 | 4 |
| 5.0 | 340 | 10 $\mu$L | 5.1 / 5.2 | 18 / 15 | 4.8 | 13 |
| 33.3 | 340 | 10 $\mu$L | 35.7 / 36.7 | 18 / 14 | 32.2 | 23 |
| 11.1 | 340 | 10 $\mu$L | 10.9 | 19 / . | 12.2 | 29 |
| 33.3 | 17 | 10 $\mu$L | 35.3 / 36.2 | <2 / <2 | 33.6 | 5 |
| 5.5 | 17 | 10 $\mu$L | 5.9 / 6.0 | <2 / <2 | 6.5 | 4 |
| 11.1 | 17 | 10 $\mu$L | 11.1 | <2 | 12.5 | 2 |

Table VIII shows the corresponding data for serum cholesterol on the enzymic yield. Again, it is clear that, after 30-40 minute incubations, the enzyme has degraded most of the bilirubin, regardless of the amount of cholesterol present.

Table VIII

| Effect of Serum Cholesterol on Citrus Enzyme Yield | | | | |
| Permutation | | | Hitachi | |
| [Cholesterol] mM | [Bu] $\mu$M | Enzyme | [Bu] $\mu$M | [chol] mM |
|---|---|---|---|---|
| 11.1 | 0 | | 1 | 10.65 |
| 5.6 | 0 | | 1 | 5.32 |
| 2.75 | 0 | | 1 | 2.7 |
| 11.1 | 0 | 5 $\mu$L | 1 | 10.7 |
| 5.6 | 0 | " | 1 | 5.35 |
| 2.75 | 0 | " | 1 | 2.68 |
| 11.1 | 170 | " | 35 | 10.3 |
| 5.6 | " | " | 35 | 5.2 |
| 2.75 | " | " | 38 | 2.6 |
| 11.1 | 17 | 0 | 19 | 10.3 |
| 5.6 | 17 | 0 | 16 | 5.3 |
| 2.75 | 17 | 0 | 15 | 2.55 |
| 11.1 | 34 | 0 | 35 | 10.4 |
| 5.6 | 34 | 0 | 36 | 5.2 |
| 2.75 | 34 | 0 | 36 | 2.56 |
| 11.1 | 17 | 5 $\mu$L | 2 | 10.1 |
| 5.6 | 17 | " | 3 | 5.3 |
| 2.75 | 17 | " | 1 | 2.55 |

It should be noted that elevated cholesterol (e.g. 11 mM or 400 mg/dL) and, to a lesser extent glucose, did slow down the initial rate of the enzymic reaction on unconjugated bilirubin by, for example, 10-20%. However, the reaction extent at for example 30-40 min. incubation was not significantly affected by either compound in a serum-like matrix. Thus, while these compounds are, enzymologically speaking, inhibitors, they do not affect the practical utility of the Citrus enzyme in removing interferences from serum due to bilirubin. Tests that are interfered severely by bilirubin include e.g. uric acid, cholesterol, glucose, triglycerides, to mention just a few (See for example, Spain and Wu, Clin. Chem. 32:518, 1986). Also, in practice, one will use much higher levels of the enzyme than those used here (5-10 $\mu$L/assay, representing about 0.1-0.2 mg protein). In such cases, the effects on initial rates will most likely be overcome or reduced to well within tolerable limits.

B. Orange Enzyme Reaction

The bilirubin-degrading assay as described under (A) was carried out using an orange enzyme preparation obtained as described in Example 1. As in the case with the lime enzyme, the reaction of the orange enzyme was largely unaffected by the presence of water-soluble serum analytes such as glucose or urea, but its initial rate was retarded (10-20%) by elevated levels of cholesterol (for example at 10mM) (Table IX). Significantly, however, is that the overall yield of the enzymic reaction was still within 90-95% of the control value which is generally acceptable in clinical diagnostics, despite the slow downs of the early kinetics. It is to be noted that the amount of enzyme used for removing interferences can be increased, if desired, to speed up the process. In summary, the results in Table IX show that the enzyme reaction yield is essentially unaffected by other serum analytes.

Table IX

| Effect of Glucose and Cholesterol on Extent of Orange Enzyme Reaction | | | | |
|---|---|---|---|---|
| **(A) Glucose** | | | | |
| **INITIAL** | | | **FINAL** | |
| Glucose mM | [Bu] $\mu$M | Enzyme $\mu$L | [Glucose] mM | [Bu] $\mu$M |
| 33 | 0 | None | 33.5 | 0 |
| 11 | 0 | " | 10.5 | 0 |
| 33 | 340 | " | 35.7 | 320 |
| 11 | 340 | " | 11.4 | 315 |
| 33 | 340 | 10 $\mu$L | 34.4 | 15 |
| 11 | 340 | 10 $\mu$L | 10.0 | 13 |
| **(B) Cholesterol** | | | | |
| **INITIAL** | | | **FINAL** | |
| Cholesterol mM | [Bu] $\mu$M | Enzyme $\mu$L | cholesterol mM | [Bu] $\mu$M |
| 10 | 0 | 0 | 11 | <2 |
| 6 | 0 | 0 | 5.7 | <2 |
| 3 | 0 | 0 | 2.8 | <2 |
| 10 | 170 | 10 | 11 | 28 |
| 6 | 170 | 10 | 5.5 | 25 |
| 3 | 170 | 10 | 2.8 | 25 |
| 10 | 17 | 10 | 9.9 | <2 |
| 6 | 17 | 10 | 5.8 | <2 |
| 3 | 17 | 10 | 3.1 | <2 |

Example 15

Effects of Common Enzyme Cofactors on the Lime Enzyme

At 1 mM final concentration, the following had no demonstrable effect on the lime enzyme preparation obtained by the procedure as described in Example 1 in the normal bilirubin-degrading assay as described in Example 2: NAD, NADH, AMP, ADP, ATP. For the orange enzyme preparation, however, 1mM ATP inhibited the activity by almost 50%.

The invention has been described in detail with particular reference to certain preferred embodiments thereof, but it will be understood that variations and modifications can be readily effected by the skilled person.

**Claims**

1. A bilirubin-specific enzyme preparation comprising an enzyme derived from a plant of the genus Citrus which, in the presence of a bilirubin-containing aqueous liquid degrades bilirubin.

2. An enzyme preparation according to claim 1 which does not generate hydrogen peroxide or biliverdin as a stoichiometric end product.

3. An enzyme preparation according to claim 1 or claim 2 wherein the bilirubin-containing aqueous liquid has a pH of about 7.5 and a temperature of about 25° C and wherein 0.022 to 0.069 micromoles of bilirubin is degraded per minute per milligram of protein.

4. An enzyme preparation according to any one of claims 1 to 3 and which after heating at a pH of about 7.5 and a temperature of about 100° C for at least 30 minutes retains about 90% of its original activity.

5. An enzyme preparation according to any one of the preceding claims and which has a Km in the range of about 50 to 270 μM bilirubin.

6. An enzyme preparation according to any one of the preceding claims wherein the enzyme is derived from limes (Citrus aurantifolia), orange (Citrus sinensis), tangerines (Citrus reticulata), tangelos, grapefruits (Citrus paradisi) or lemons (Citrus limon).

7. An enzyme preparation according to any one of the preceding claims wherein the enzyme is derived from the peel of limes, oranges, tangerines, tangelos, grapefruits or lemons.

8. A bilirubin-specific enzyme preparation comprising an enzyme derived from limes (Citrus aurantifolia) which, in the presence of a bilirubin-containing aqueous liquid having a pH of about 7.5 and a temperature of about 25° C degrades 0.035 to 0.069 micromoles of bilirubin per minute per milligram of protein.

9. A bilirubin-specific enzyme preparation comprising an enzyme derived from limes (Citrus aurantifolia) which, at a pH of about 5.0 degrades a tauro-conjugate of bilirubin, conjugated bilirubin, or delta bilirubin.

10. A bilirubin-specific enzyme preparation as claimed in claim 8 or claim 9, wherein the enzyme is derived from the peel of limes.

11. An assay composition for the determination of an analyte other than bilirubin in an aqueous liquid sample, comprising an interactive composition and a bilirubin-specific enzyme preparation according to any one of claims 1 to 10 whereby bilirubin interference with the assay is reduced.

12. An analytical element for detecting an analyte, comprising a bilirubin-specific enzyme derived from a plant of the genus Citrus as defined in any one of claims 1 to 10.

13. A dry analytical element for the assay of an aqueous liquid, the element including a support and a reagent zone containing a bilirubin-specific enzyme derived from a plant of the genus Citrus as defined in any one of claims 1 to 10.

14. The element as claimed in claim 13 wherein the enzyme is present in an amount of from about 0.5 to 2.0 mg/ft$^2$.

15. A method for the degradation of bilirubin in an aqueous liquid sample comprising contacting the liquid sample with a bilirubin-specific enzyme preparation according to any one of claims 1 to 10.

16. The method as claimed in claim 15, wherein the aqueous liquid sample is a biological fluid.

17. A method for the determination of bilirubin in an aqueous liquid sample comprising:

(a) contacting the liquid sample with a bilirubin-specific enzyme preparation according to any one of claims 1 to 10, to interact bilirubin with the enzyme preparation and to produce a detectable change; and

(b) detecting the change.

18. A method for the determination of an analyte other than bilirubin in an aqueous liquid sample comprising:

(a) treating the sample with an interactive composition for the analyte to produce a detectable change;

(b) prior to or during step (a) contacting the sample with a bilirubin-specific enzyme preparation according to any one of claims 1 to 10, thereby reducing the potential of bilirubin interference with the detectable change produced in (a); and

(c) detecting the change produced in step (a).

19. A method for the determination of bilirubin in an aqueous liquid sample comprising:

(a) contacting the liquid sample with an analytical element including a support and a reagent zone containing a bilirubin-specific enzyme derived from a plant of the genus Citrus as defined in any one of claims 1 to 10, to interact bilirubin with the enzyme and to produce a detectable change; and

(b) detecting the change.

20. The method as claimed in claim 19, wherein the detectable change is a change in the absorption density, fluorescence intensity or other physical change accompanying the degradation of bilirubin.

21. A method for separating bilirubin at acidic pH using the bilirubin-specific enzyme preparation according to claim 8 or claim 9.

Absorbance

1.0

0

30

FIG. 1a

400    500    600    700

nM

Absorbance

1.0

Bu alone

0

2

4

10

15

Enzyme
alone

FIG. 1b

400    500    600    700

nM

EP 0 320 095 A2

FIG. 2

FIG.3

FIG.4a

FIG.4b

EP 0 320 095 A2

FIG.5

EP 0 320 095 A2

FIG.6

EP 0 320 095 A2